## Europäisches Patentamt
## European Patent Office
## Office européen des brevets

(19)

(11) Veröffentlichungsnummer: **0 064 212**
**B1**

(12) # EUROPÄISCHE PATENTSCHRIFT

(45) Veröffentlichungstag der Patentschrift:
**13.07.88**

(51) Int. Cl.⁴: **A 61 M 25/00**

(21) Anmeldenummer: **82103285.1**

(22) Anmeldetag: **20.04.82**

(54) **Katheterbesteck.**

(30) Priorität: **06.05.81 DE 3117802**

(43) Veröffentlichungstag der Anmeldung:
**10.11.82 Patentblatt 82/45**

(45) Bekanntmachung des Hinweises auf die Patenterteilung:
**13.07.88 Patentblatt 88/28**

(84) Benannte Vertragsstaaten:
**AT BE CH DE FR GB IT LI LU NL SE**

(56) Entgegenhaltungen:
**DE - A - 2 340 534**
**DE - A - 3 107 983**
**DE - B - 1 087 323**
**DE - U - 7 427 426**
**GB - A - 2 033 236**
**US - A - 3 459 184**
**US - A - 3 537 452**
**US - A - 3 570 485**
**US - A - 3 633 579**
**US - A - 4 239 042**
**US - A - 4 243 050**

(73) Patentinhaber: **INTERMEDICAT GMBH,
Gerliswilstrasse 43, CH-6020 Emmenbrücke (CH)**

(72) Erfinder: **Weikl, Andreas, Dr., Kosbacher Weg 51,
D-8520 Erlangen (DE)**
Erfinder: **Hubmann, Max, Dr., Ratsberger Strasse 24,
D-8520 Erlangen (DE)**

(74) Vertreter: **von Kreisler, Alek, Dipl.-Chem. et al,
Deichmannhaus am Hauptbahnhof, D-5000 Köln 1 (DE)**

## Beschreibung

Die Erfindung bezieht sich auf ein Katheterbesteck gemäss dem ersten Teil des Patentanspruchs 1. Ein derartiges Katheterbesteck ist bekannt durch US-A-3 570 485.

Bei dem erwähnten bekannten Katheterbesteck ist eine flexible, schlauchförmige Kathetereinführröhre vorgesehen, die nach der Punktion eines Blutgefässes von der Gefässwand und dem umgebenden Gewebe zusammengedrückt wird. Wird in die Kathetereinführröhre ein Katheter eingeschoben, so weitet er den zusammengedrückten Abschnitt der Kathetereinführröhre auf, bevor er in das Blutgefäss eindringt. Die Kathetereinführröhre ist mit einem Längsschlitz versehen, so dass sie nach dem Herausziehen aus dem Blutgefäss unter Zurücklassung des Katheters gesplittet und seitlich von dem Katheter entfernt werden kann. Die Punktionsstelle schliesst sich also um den Katheter herum, so dass seitlich vom Katheter kein Blut austreten kann. Voraussetzung für die Funktion eines derartigen Katheterbestecks ist, dass die Kathetereinführröhre aus einem weichen Material besteht. Eine solche weiche Kathetereinführröhre ist aber zum Führen eines Katheters nicht besonders gut geeignet.

Durch DE-A-2 340 534 ist ferner ein Katheterbesteck mit einer Y-förmigen Kathetereinführröhre bekannt. Die Punktionskanüle wird in einen gradlinigen Kanal der Kathetereinführröhre eingeschoben und nach erfolgter Punktion herausgezogen. In den Abzweig der Kathetereinführröhre wird ein flexibler Katheter eingeschoben. Die Kathetereinführröhre ist auch hier gesplittet, d.h. sie weist eine durchgehende Längsfuge auf, um durch Aufspreizen seitlich von dem Katheter entfernt werden zu können.

Der Erfindung liegt die Aufgabe zugrunde, ein Katheterbesteck der eingangs erwähnten Art zu schaffen, bei dem der einzuführende Katheter seinen Weg in und durch das Blutgefäss findet, obwohl die Kathetereinführröhre aus einem relativ starren Material bestehen kann.

Diese Aufgabe wird erfindungsgemäss durch die kennzeichnenden Merkmale des Patentanspruchs 1 gelöst.

Nach der Erfindung weist der Katheter einen als Führungsglied dienenden rüsselförmigen Abschnitt auf, der durch den vorderen Bereich der Kathetereinführröhre hindurchgeschoben wird, ohne diesen vorderen Bereich aufzuweiten. Erst wenn der Bereich grösseren Aussendurchmessers des Katheters den vorderen Bereich der Kathetereinführröhre passiert, wird dieser vordere Bereich aufgeweitet. Zu dieser Zeit befindet sich der rüsselförmige Abschnitt bereits im Inneren des Blutgefässes, um den Weg des Katheters vorzugeben. Mit dem erfindungsgemässen Katheterbesteck lassen sich Überdehnungen des Blutgefässes oder Einrisse desselben an oder im Bereich der Punktionsstelle beim Einführen des Katheters vermeiden. Es ist also mit dem erfindungsgemässen Katheterbesteck nicht nur eine sichere Abdichtung der Punktionsstelle gewährleistet, sondern es werden auch die bisher so störenden Nebenfolgen, wie das Eindringen von Luft, Schmutzpartikeln oder dergleichen in die Blutbahn mit wesentlich höherer Sicherheit ausgeschlossen.

Vorteilhafte weitere Ausgestaltungen des Erfindungsgedankens sind Gegenstand von Unteransprüchen.

Die Erfindung wird nachfolgend anhand von Ausführungsbeispielen näher erläutert, die in vergrössertem Massstab in der Zeichnung dargestellt sind.

Es zeigen:

Fig. 1 und Fig. 2 ein Ausführungsbeispiel eines vollständigen Set des Katheterbesteckes, wobei in Fig. 1 die Einführhülse mit der Kanüle und in Fig. 2 der Katheter mit Mandrin dargestellt ist,

Fig. 3 bis Fig. 8 die verschiedenen Stufen bei der Benutzung des Katheterbesteckes,

Fig. 9 einen Querschnitt des Aufweitungsbereichs einer Kathetereinführröhre mit zur Längsachse parallelen, innenliegenden Schwächungszonen,

Fig. 10 einen Querschnitt des Aufweitungsbereichs einer Kathetereinführröhre mit zur Längsachse parallelen Stabilisierungselementen und innenliegenden Schwächungszonen,

Fig. 11 einen Querschnitt einer Kathetereinführröhre mit zur Längsachse parallelen diskreten Aufweitungsbereichen aus gummielastischem Material,

Fig. 12 und Fig. 13 einen Querschnitt des Aufweitungsbereichs einer Kathetereinführröhre mit einer durchgehenden Längstrennstelle parallel zur Längsachse, wobei die Enden der Kathetereinführröhre im Bereich der Längstrennstelle mit Anschäftungen versehen sind, jeweils im Querschnitt in der Normallage und in der durch den Katheter aufgeweiteten Stellung und

Fig. 14 und Fig. 15 einen Querschnitt des Aufweitungsbereichs einer Kathetereinführröhre mit einer durchgehenden Längstrennstelle parallel zur Längsachse, wobei die Enden der Kathetereinführröhre mit zueinander korrespondierenden stufenförmigen Absätzen versehen sind, jeweils im Querschnitt in der Normallage und in der durch den Katheter aufgeweiteten Stellung.

Das erfindungsgemässe Katheterbesteck besteht aus einer üblichen Punktionskanüle 30 aus Stahl, die von einer Kathetereinführröhre 1 koaxial umgeben ist. Letztere wird zusammen mit der vorgenannten Punktionskanüle durch die Haut in ein Blutgefäss 3 eingeführt, wobei die Punktionskanüle anschliessend durch Zurückziehen ganz aus der Kathetereinführröhre 1 entfernt wird. Anschliessend wird der Katheter 4 mit dem koaxialen Mandrin 5 in die Kathetereinführröhre 1 eingeführt. Die Kathetereinführröhre 1 ist an ihrem in das Blutgefäss 3 einzuführenden Ende leicht konisch verjüngt. Der Aussendurchmesser der Kathetereinführröhre 1 im Bereich der Punktionsstelle 3a ist gleich oder wenigstens angenähert gleich dem Aussendurchmesser am gefässseitigen Ende der Kathetereinführröhre 1.

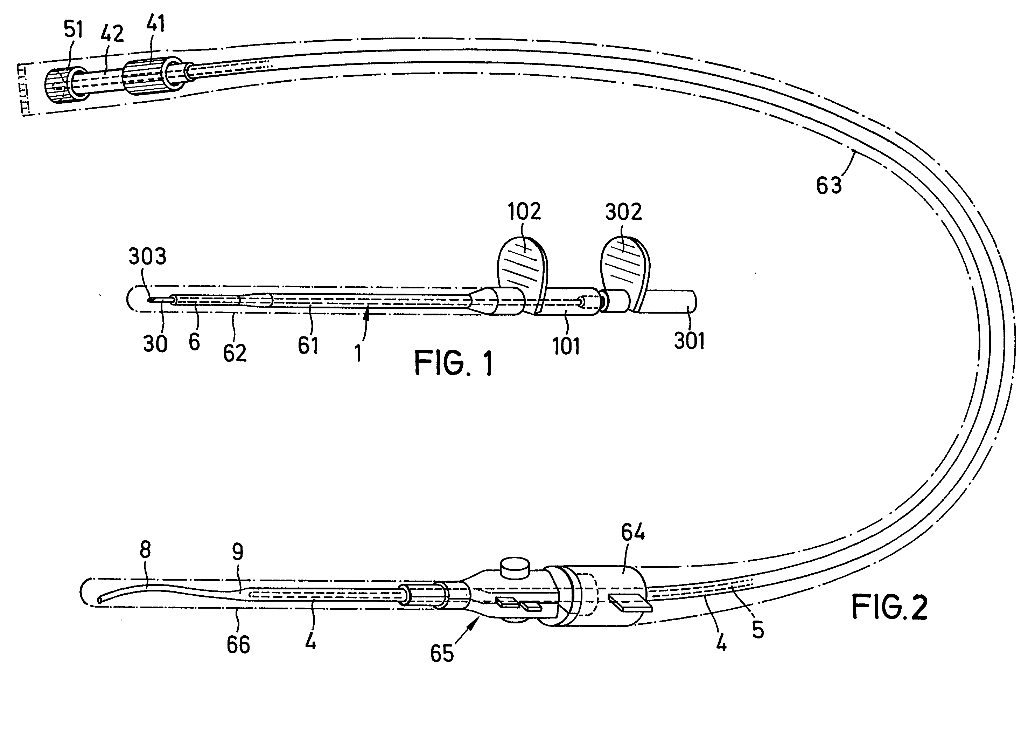

In Fig. 1 ist eine Punktionskanüle 30 dargestellt, die aus einem Stahlrohr besteht, das an seinem rückwärtigen Ende einen rohrförmigen Kunststoffansatz 301 mit einem abstehenden Griffstück 302 aufweist. Das vordere Ende 303 der Punktionskanüle 30 ist zum Einstechen in die Haut schräg angeschliffen. Über die Punktionskanüle 30 ist die Kathetereinführröhre 1 geschoben. Die Kathetereinführröhre 1 weist an ihrem vorderen Ende einen aufweitbaren Abschnitt 6 auf, dessen Innendurchmesser dem Aussendurchmesser der Punktionskanüle 30 entspricht und an den sich ein zylindrischer Abschnitt 61 mit grösserem Durchmesser anschliesst. An dem rückwärtigen Ende des Abschnittes 61 ist ein rohrförmiger Ansatz 101 aus Kunststoff befestigt, der ein seitlich abstehendes Griffstück 102 aufweist.

Die Punktionskanüle 30 ist durch die Längsbohrung des Kunststoffansatzes 101 und durch die Kathetereinführröhre 1 hindurchgeschoben und ragt am vorderen Ende aus der Kathetereinführröhre heraus. Zum Schutz gegen Verletzungen ist eine rohrförmige Kunststoffkappe 62 vorgesehen, die den vorderen Bereich der Kanüle 30 und die Kathetereinführröhre 1 überdeckt und klemmend auf das vordere Ende des Kunststoffansatzes 101 aufgeschoben ist.

Der Katheter 4 mit dem in ihn eingelegten Mandrin 5 ist in Fig. 2 dargestellt. Der Katheter 4 besteht aus einem rohrförmigen Schlauch, dessen vorderer Abschnitt 8 einen kleineren Durchmesser hat. Der durch den Katheter 4 hindurchführende Kanal setzt sich in dem vorderen Abschnitt 8 mit kleinerem Durchmesser fort, d.h. der vordere Abschnitt 8 des Katheters 4 ist ebenfalls hohl. Der in den Katheter 4 eingeschobene Mandrin 5 ragt mit seinem vorderen Ende bis zu der Stelle 9, an der der verjüngte Katheterabschnitt 8 beginnt.

Am rückwärtigen Ende des Katheters 4 befindet sich ein aus Kunststoff bestehendes rohrförmiges Anschlussstück 42, auf dem ein rohrförmiges Kupplungsstück 41 drehbar angeordnet ist. An dem rückwärtigen Ende des Mandrins 5 ist ein Anschlussstück 51 befestigt, das mit dem Anschlussstück 42 des Katheters 4 verriegelbar ist, so dass die rückwärtigen Enden von Katheter 4 und Mandrin 5 relativ zueinander fixierbar sind.

Der Katheter 4 ist von einem aus einer durchsichtigen dünnen Kunststoffolie bestehenden Schutzschlauch 63 umgeben, um die Sterilität des Katheters zu gewährleisten. Am vorderen Ende des Schutzschlauches 63 ist ein rohrförmiges Anschlussstück 64 befestigt. Dieses Anschlussstück 64 ist auf das rückwärtige Ende eines Einführstückes 65 aufgeschoben. Dieses Einführstück besteht aus zwei Hälften, die an einer Längsseite scharnierartig miteinander verbunden sind und aufgeklappt werden können. Durch das Einführstück 65 wird der Katheter 4 hindurchgeschoben, wobei der Schutzschlauch 63 sich flexibel zusammenlegt. Gemäss Fig. 2 ist auf das vordere Ende des Einführstückes 65 eine Schutzhaube 66 aus Kunststoff aufgeschoben, die das vordere Ende des Katheters 4 gegen Kontamination schützt.

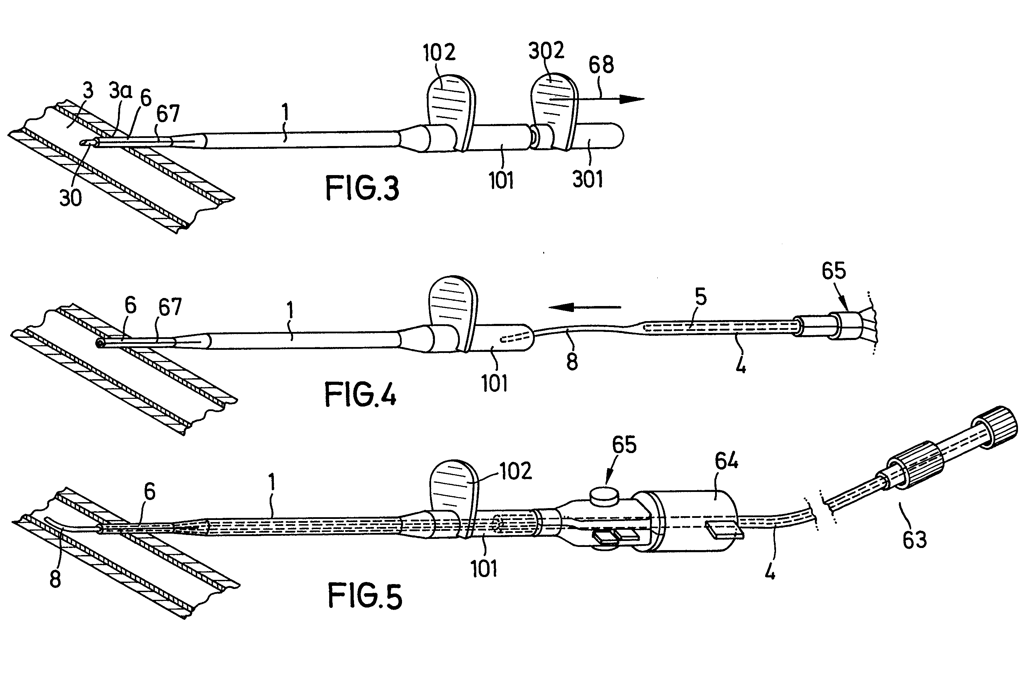

Fig. 3 zeigt das Einstechen der Punktionskanüle 30 mit darüber geschobener Kathetereinführröhre 1 in ein Blutgefäss 3. Die Kathetereinführröhre 1 besteht aus dünnwandigem starren Kunststoff. Der Ausweitungsbereich 6 weist einen Längsschlitz 67 auf, so dass er später aufgeweitet werden kann. Nachdem die Kanüle mit der Kathetereinführröhre 1 in der in Fig. 3 dargestellten Weise in das Blutgefäss 3 eingestochen worden ist, wird die Punktionskanüle 30 durch Zurückziehen des Griffstückes 302 in Richtung des Pfeiles 68 entfernt, während die Kathetereinführröhre 1 in ihrer Position bleibt. Danach wird gemäss Fig. 4 der Katheter 4 aus dem Einführstück 65 heraus durch die Bohrung des Ansatzes 101 hindurch in die Kathetereinführröhre 1 eingeschoben, bis der weiche vordere Abschnitt 8 des Katheters 4 gemäss Fig. 5 in das Blutgefäss 3 eindringt und von diesem geführt werden kann. Nun wird das vordere Ende des Einführstückes 65 gemäss Fig. 5 in den Ansatz 101 der Kathetereinführröhre 1 eingeschoben, so dass der Katheter 4, ohne mit der Hand berührt zu werden, aus dem Einführstück 65 in die Kathetereinführröhre 1 gleitet. Das Vorschieben des Katheters 4 geschieht dadurch, dass der Katheter 4 durch Anfassen an dem Folienschlauch 63 bewegt wird.

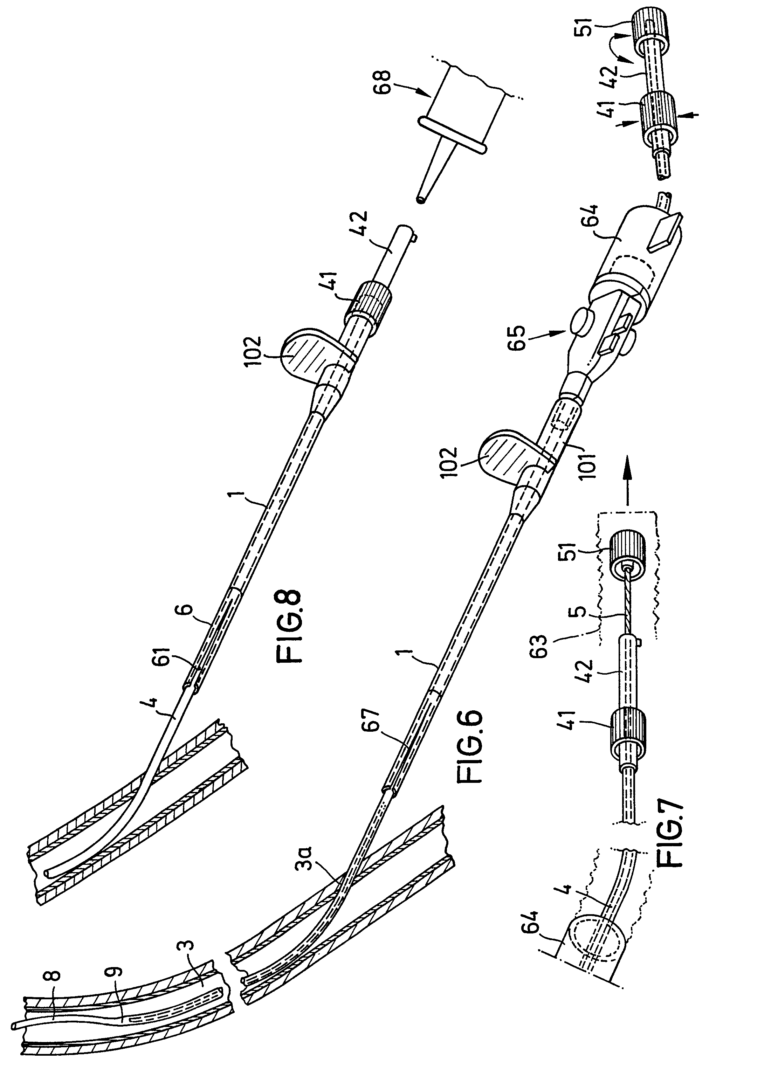

Wenn die Stelle 9 des Katheters 4 den verjüngten Aufweitungsbereich 6 der Kathetereinführröhre 1 passiert, wird der Schlitz 67 auseinandergedrückt. Gleichzeitig wird die Kathetereinführröhre 1 gemäss Fig. 6 zurückgezogen, während der Katheter 4 weiter vorgeschoben wird.

Nachdem der Katheter 4 in dem Blutgefäss 3 verlegt worden ist, wird der Mandrin 5 durch Ziehen an dem Anschlussstück 51 aus dem Katheter 4 herausgezogen. Gleichzeitig wird der Folienschlauch 63 mit dem Ring 64 nach hinten abgezogen.

Dann wird gemäss Fig. 8 das rückwärtige Ende des Katheters 4 mit dem rückwärtigen Ende der Kathetereinführröhre 1 verbunden, indem das Kupplungsstück 41 auf den Ansatz 101 aufgeschraubt wird. Nun kann durch Einsetzen des vorderen Endes einer Spritze 68 in den Ansatz 42 Flüssigkeit in den Katheter 4 injiziert werden.

Der rüsselförmige Abschnitt 8 des Katheters 4 muss nicht unbedingt einstückig mit dem Katheter 4 ausgebildet sein. Der Abschnitt 8 kann auch auf ein entsprechendes Ansatzstück des Katheters 4 aufgesetzt und mit diesem fest verbunden werden. Auch ist es möglich, den Abschnitt 8 koaxial in den Katheter 4 einzuführen, wobei bei dieser Ausführungsform dann unterschiedliche Materialien zur Optimierung der Funktion einerseits als Führungsglied und andererseits als Dehnungselement für die zurückführende Kathetereinführröhre 1 und als Abdichtung der Punktionsstelle 3a verwendet werden können.

Der Katheter 4 und der Mandrin 5 bestehen vorzugsweise aus abriebfestem, ausreichend flexiblem Kunststoff, um einerseits den unbeabsichtigten Abrieb auch kleinster Kunststoffpartikel zu vermeiden und um andererseits diese beiden Teile sicher und gefahrlos in dem Blutgefäss 3 plazieren zu können.

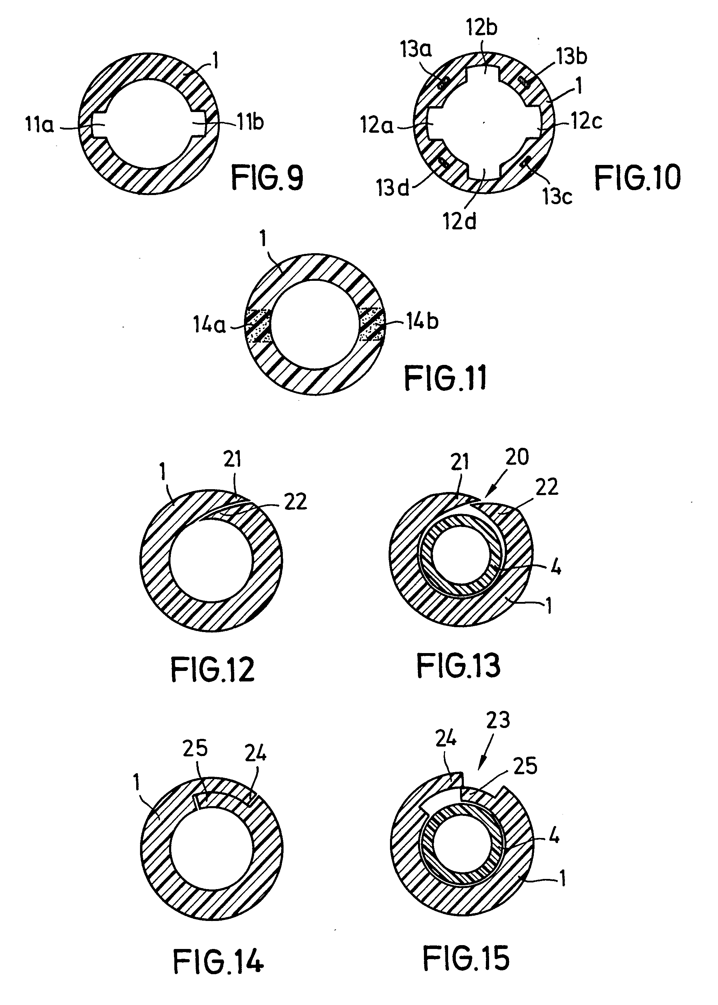

Wie die Fig. 9 zeigt, kann der Aufweitungsbereich aus diskreten Dehnsegmenten aus Zonen 11a und 11b mit verringerter Materialstärke bestehen. Diese Schwächungszonen 11a, 11b verlaufen vorzugsweise parallel zur Längsachse und stellen Erweiterungen des Öffnungsquerschnittes der Kathetereinführröhre 1 dar. Da die Wandstärke der Kathetereinführröhre 1 gering ist und beispielsweise bei 0,1 bis 0,3 mm liegt, lässt sich bereits durch geringfügige Reduzierung der Materialstärke in den Zonen 11a, 11b eine ausreichende Dehnung erzielen. Dies gilt insbesondere für ausreichend elastische Materialien für die Kathetereinführröhre 1.

Um der Kathetereinführröhre 1 trotz Verwendung eines ausreichend elastischen Materials eine ausreichende Formsteifigkeit zu geben, um ein sicheres Einführen in die Punktionsstelle 3a zu gewährleisten, kann die Kathetereinführröhre 1 gemäss Fig. 10 an ihrem Umfang mit mindestens einem, vorzugsweise aber mit mehreren, der Anzahl der Schwächungszonen 12a–12d entsprechenden Stabilisierungselemente 13a–13d versehen sein. Diese Stabilisierungselemente 13a–13d verlaufen vorzugsweise parallel zur Längsachse der Kathetereinführröhre 1 und bestehen vorzugsweise aus federelastischem Material, insbesondere Federstahl. Diese Stabilisierungselemente werden im Zuge der Herstellung der Kathetereinführröhre 1 gleich in die materialmässig nicht verdünnten Zonen der Kathetereinführröhre 1 eingebettet.

Wie Fig. 11 zeigt, kann die Kathetereinführröhre 1 auch Dehnungssegmente 14a und 14b aus einem Material mit besonders elastischen Eigenschaften, beispielsweise Silikongummi oder natürlicher Kautschuk, aufweisen. Zur besseren Führung der Kathetereinführröhre 1 kann diese an dem der Punktionsstelle 3a abgewandten Ende mit einem Haltegriff versehen sein.

Die Fig. 12 und 13 zeigen Ausführungsformen der Kathetereinführröhre 1 mit einer zur Längsachse parallelen, durchgehenden Längstrennstelle 20, wobei die Fig. 12 die Kathetereinführröhre 1 im Ausgangszustand ohne eingeführten Katheter 4 zeigt. Die Ränder 21, 22 der Längstrennstelle 20 der Kathetereinführröhre 1 sind im Bereich der Längstrennstelle 20 mit korrespondierenden Schrägflächen versehen, damit auch im aufgeweiteten Zustand noch eine geringfügige Überlappung der Ränder 21, 22 der Längstrennstelle 20 der Kathetereinführröhre 1 gegeben ist.

Die Fig. 14 und 15 zeigen eine andere Variante mit einer zur Längsachse parallel verlaufenden, durchgehenden Längstrennstelle 23. In diesem Falle sind die Ränder 24, 25 der Längstrennstelle 23 der Kathetereinführröhre 1 im Bereich der Längstrennstelle 23 mit korrespondierenden stufenförmigen Absätzen versehen. Auch bei dieser Ausführungsform wird also noch eine gewisse Überlappung der stufenförmigen Absätze 24, 25 im aufgeweiteten Zustand der Kathetereinführröhre 1 sichergestellt.

Mit der Erfindung ist ein Katheterbesteck geschaffen worden, das sich nicht nur einfach und sicher handhaben lässt, sondern das auch eine absolut wirksame Abdichtung der Punktionsstelle gewährleistet, so dass die vielfältigen Nachteile, die eine leckende Punktionsstelle mit sich bringt, sicher vermieden sind.

Mit dem erfindungsgemässen Katheterbesteck liegt ein Einführungsbesteck nach der Seldinger-Technik vor, das lediglich aus zwei gesondert zu handhabenden Teilen, nämlich der Kathetereinführröhre und dem Katheter bzw. dem Bausatz aus Katheter und Mandrin besteht. Damit wird die Handhabung dieses Bestecks wesentlich vereinfacht und sicherer als dies bei der bisher praktizierten Seldinger-Technik der Fall ist. Ausserdem bietet dieses Katheterbesteck den Vorteil, dass die Bedingungen der Asepsis wesentlich einfacher einzuhalten sind. Vorteilhaft ist auch die Möglichkeit, dieses erfindungsgemässe Katheterbesteck mit einem automatisch verschliess- und verriegelbaren Katheteranschlusskopf zu kombinieren, so dass ein komplettes, einteiliges Besteck nach der Seldinger-Technik erhalten werden kann.

**Patentansprüche**

1. Katheterbesteck bestehend aus einem Katheter (4), einer Punktionskanüle (30) und einer koaxial zur Punktionskanüle (30) angeordneten, nach dem Herausziehen der Punktionskanüle zum Einführen des Katheters (4) in ein Blutgefäss (3) dienenden Kathetereinführröhre (1), die einen sich in Längsrichtung erstreckenden, eine Durchmesserveränderung zulassenden Verformungsbereich (6) aufweist, derart, dass die Kathetereinführröhre (1) über einen Teil ihrer Länge durch den Katheter (4) aufweitbar ist und die Wand des Blutgefässes (3) den Katheter an der Punktionsstelle (3a) nach dem Herausziehen der Kathetereinführröhre (1) eng umschliesst, dadurch gekennzeichnet, dass der am vorderen Ende der Kathetereinführröhre (1) angeordnete Verformungsbereich ein Aufweitungsbereich (6) ist, an den sich nach hinten ein durch den Katheter (4) nicht aufweitbarer zylindrischer Katheterführungsabschnitt (61) mit grösserem Innendurchmesser anschliesst, und dass der Katheter (4) mit einem als Führungsglied dienenden rüsselförmigen Abschnitt (8) mit konstantem, gegenüber dem Katheter (4) verringerten Aussendurchmesser versehen ist, wobei zwischen dem rüsselförmigen Abschnitt (8) und dem Katheter (4) ein konischer Übergangsbereich (9) vorgesehen ist, der beim Einführen des Katheters (4) in die Kathetereinführröhre (1) deren Aufweitungsbereich (6) auseinanderdrückt, während sich das vordere Ende des rüsselförmigen Abschnitts (8) bereits im Blutgefäss (3) befindet.

2. Katheterbesteck nach Anspruch 1 oder 2, dadurch gekennzeichnet, dass der Aufweitungsbereich (6) aus diskreten Aufweitungssegmenten besteht.

3. Katheterbesteck nach Anspruch 2, dadurch gekennzeichnet, dass die Aufweitungssegmente Dehnsegmente (14a, 14b) aus gummielastischem Material sind.

4. Katheterbesteck nach Anspruch 2, dadurch gekennzeichnet, dass die diskreten Aufweitungssegmente (11a, 11b; 12a–12d) aus Zonen mit verringerter Materialstärke bestehen (Fig. 9; 10).

5. Katheterbesteck nach Anspruch 2, dadurch gekennzeichnet, dass ein einziges Aufweitungssegment vorhanden ist, das aus einer zur Längsachse parallelen Längstrennstelle (20, 23) besteht (Fig. 12, 13; 14, 15).

6. Katheterbesteck nach Anspruch 5, dadurch gekennzeichnet, dass die Ränder (21, 22) der Längstrennstelle (20) mit korrespondierenden Schrägflächen versehen sind (Fig. 12, 13).

7. Katheterbesteck nach Anspruch 5, dadurch gekennzeichnet, dass die Ränder (24, 25) der Längstrennstelle (23) mit korrespondierenden stufenförmigen Absätzen versehen sind (Fig. 14; 15).

8. Katheterbesteck nach einem der Ansprüche 1 bis 7, dadurch gekennzeichnet, dass der Öffnungsquerschnitt des rüsselförmigen Abschnitts (8) gleich dem Öffnungsquerschnitt im Katheter (4) ist.

### Claims

1. Catheter set comprising a catheter (4), a puncturing canula (30) and a catheter insertion tube (1) arranged coaxially to the puncturing canula (30) and serving, upon the removal of the puncturing canula for the introduction of the catheter (4) into a blood vessel (3), said tube (1) having a deformable area (6) extending in longitudinal direction and allowing a change of the diameter of the deformable area (6) so that the catheter insertion tube (1) is dilatable over part of its length by the catheter (4) and that the wall of the blood vessel (3) tightly encloses the catheter at the puncture point (3a) upon the retraction of the catheter insertion tube (1), characterized in that the deformable area provided at the front end of the catheter insertion tube (1) is a dilatable area (6) followed rearwardly by a cylindrical catheter guide section (61) non dilatable by the catheter (4) and having a larger inner diameter and that the catheter (4) is provided with a trunk-shaped section (8) serving as a guide member and having a constant outer diameter reduced with respect to the catheter (4), a conical transitional region (9) being provided between the trunk-shaped section (8) and the catheter (4) which, upon the introduction of the catheter (4) into the catheter insertion tube (1) presses its dilatable area (6) apart while the front end of the trunk-shaped section (8) is already in the blood vessel (3).

2. Catheter set as defined in claim 1, characterized in that the dilatable area (6) consists of discrete expansion segments.

3. Catheter set as defined in claim 2, characterized in that the expansion segments are dilatable segments (14a, 14b) of rubber-elastic material.

4. Catheter set as defined in claim 2, characterized in that the discrete expansion segments (11a, 11b; 12a–12d) consist of zones of a reduced material thickness (Fig. 9; 10).

5. Catheter set as defined in claim 2, characterized in that one sole expansion segment is provided which consists of a longitudinal separation area (20, 23) parallel to the longitudinal axis (Fig. 12, 13; 14, 15).

6. Catheter set as defined in claim 5, characterized in that the edges (21, 22) of the longitudinal separation area (20) are provided with corresponding oblique surfaces (Fig. 12, 13).

7. Catheter set as defined in claim 5, characterized in that the edges (24, 25) of the longitudinal separation area (23) are provided with corresponding step-like portions (Fig. 14: 15).

8. Catheter set as defined in one of claims 1 to 7, characterized in that the opening cross section of the trunk-shaped section (8) is equal to the opening cross section in the catheter (4).

### Revendications

1. Jeu de cathéter constitué par un cathéter (4), une canule de ponction (30) et un tube (1) d'insertion du cathéter, qui est disposé coaxialement à la canule de ponction (30), sert à introduire le cathéter (4) dans un vaisseau sanguin (3) après le retrait de la canule de ponction, et comporte une zone de déformation (6) qui s'étend dans la direction longitudinale et autorise une variation du diamètre de sorte que le tube (1) d'insertion du cathéter peut être élargi sur une partie de sa longueur, par le cathéter (4) et que la paroi du vaisseau sanguin (3) s'applique étroitement sur le cathéter, au niveau du point de ponction (3a), une fois que le tube (1) d'insertion du cathéter a été retiré, caractérisé en ce que la zone de déformation située sur l'extrémité avant du tube (1) d'insertion du cathéter est une zone d'élargissement (6), à laquelle se raccorde, vers l'arrière, une section cylindrique (61) de guidage du cathéter, qui ne peut pas être élargie par le cathéter (4) et possède un diamètre intérieur supérieur, et en ce que le cathéter (4) comporte une section en forme de trompe (8) utilisée comme organe de guidage et possédant un diamètre extérieur constant réduit par rapport à celui du cathéter (4), tandis qu'entre la section en forme de trompe (8) et le cathéter (4) il est prévu une zone de jonction conique (9) qui, lors de l'introduction du cathéter (4) dans le tube (1) d'insertion du cathéter, élargit la zone d'élargissement (6), alors que l'extrémité avant de la section en forme de trompe (8) est déjà située dans le vaisseau sanguin (3).

2. Jeu de cathéter selon la revendication 1 ou 2, caractérisé en ce que la zone d'élargissement (6) est constituée par des segments d'élargissement discrets.

3. Jeu de cathéter selon la revendication 2, caractérisé en ce que les segments d'élargissement sont des segments extensibles (14a, 14b) réalisés en un matériau présentant l'élasticité du caoutchouc.

4. Jeu de cathéter selon la revendication 2, caractérisé en ce que les segments d'élargissement discrets (11a, 11b; 12a–12d) sont constitués par des zones, dont le matériau possède une épaisseur réduite (figures 9; 10).

5. Jeu de cathéter selon la revendication 2, caractérisé en ce qu'il est prévu un seul segment d'élargissement, qui est constitué par une zone de séparation longitudinale (20, 23) parallèle à l'axe longitudinal (figures 12, 13; 14, 15).

6. Jeu de cathéter selon la revendication 5, caractérisé en ce que les bords (21, 22) de la zone de séparation longitudinale (20) comportent des surfaces obliques correspondantes (figures 12, 13).

7. Jeu de catheter selon la revendication 5, caractérisé en ce que les bords (24, 25) de la zone de séparation longitudinale (23) comportent des parties étagées qui se correspondent (figures 14; 15).

8. Jeu de cathéter selon l'une des revendications 1 à 7, caractérisé en ce que la section transversale de l'ouverture de la section en forme de trompe (8) est égale à la section transversale de l'ouverture présente dans le cathéter (4).

FIG. 1

FIG. 2

FIG.3

FIG.4

FIG.5

FIG.8

FIG.6

FIG.7

0 064 212

FIG.9

FIG.10

FIG.11

FIG.12

FIG.13

FIG.14

FIG.15